# EUROPEAN PATENT SPECIFICATION

(11) **EP 2 523 717 B1**
(45) Date of publication and mention of the grant of the patent: **25.11.2015**
(21) Application number: 10813029.5
(22) Date of filing: 17.12.2010
(51) Int. Cl.: A61M 16/06

(54) **REPLACEABLE NASAL PILLOW KIT**
AUSTAUSCHBARES NASENMASKENKIT
KIT D'EMBOUTS NASAUX REMPLAÇABLES

(30) Priority: 15.01.2010 US 295253 P
(43) Date of publication of application: 21.11.2012
(73) Proprietor: Koninklijke Philips N.V., 5656 AE Eindhoven (NL)
(72) Inventor: HO, Peter Chi Fai, Briarcliff Manor, NY 10510-8001 (US)
(74) Representative: van Velzen, Maaike Mathilde
(86) International application number: PCT/IB2010/055921
(87) International publication number: WO 2011/086437

(56) References cited:
- WO-A1-2005/063328
- WO-A1-2008/011682
- WO-A1-2008/014543
- US-A1- 2008 289 633

## Description

The present invention pertains to patient interface devices, and, in particular, to patient interface devices having replaceable nasal pillows. The invention further pertains to kits providing replaceable nasal pillows.

There are numerous situations where it is necessary or desirable to deliver a flow of breathing gas non-invasively to the airway of a patient, i.e., without intubating the patient or surgically inserting a tracheal tube in the esophagus. For example, it is known to ventilate a patient using a technique known as non-invasive ventilation. It is also known to deliver continuous positive airway pressure (CPAP) or variable airway pressure, such as a bi-level pressure that varies with the patient's respiratory cycle or an auto-titrating pressure that varies with the monitored condition of the patient. Typical pressure support therapies are provided to treat a medical disorder, such as sleep apnea syndrome, in particular, obstructive sleep apnea (OSA), or congestive heart failure.

Non-invasive ventilation and pressure support therapies involve the placement of a patient interface device, which is typically a nasal or nasal/oral mask, on the face of a patient to interface the ventilator or pressure support system with the airway of the patient so that a flow of breathing gas can be delivered from the pressure/flow generating device to the airway of the patient. It is known to maintain such masks on the face of a patient by a headgear having upper and lower straps, each having opposite ends threaded through connecting elements provided on the opposite sides and top of a mask.

Because such masks are typically worn for an extended period of time, a variety of concerns must be taken into consideration. For example, in providing CPAP to treat OSA, the patient normally wears the patient interface device all night long while he or she sleeps. One concern in such a situation is that the patient interface device is as comfortable as possible, otherwise the patient may avoid wearing the interface device, defeating the purpose of the prescribed pressure support therapy. It is also important that the interface device provides a tight enough seal against a patient without discomfort.

In general, patients using a mask interface under ventilation require a specifically sized mask to fit the dimensions of the patients facial features. This is also true for patients who use nasal pillows where sizing is less obvious, and therefore can more commonly occur, than in applications where a conventional triangular interface is used. Providers generally attempt to address fitment problems by giving the patient a number of masks with various sizes to pick from. Such solution can become quite cumbersome in application involving multiple large size masks and confusing. Such solution also fails to address a further issue of patients with asymmetric nostrils. Further known systems are disclosed in WO 2005/063328.

Accordingly, it is an object of the present invention to provide a patient interface device that overcomes the shortcomings of conventional patient interface devices. This object is achieved by the features of claim 1. An example provides a kit for fitting a patient interface device to the naris of a patient for use with a patient interface device employing a replaceable nasal pillow. The kit comprises a plurality of nasal pillows. Each of the nasal pillows comprises a base portion, a casing, and a fill material. The base portion is structured to be disposed on a portion of the patient interface device. The casing is coupled to the base in a manner which defines a scaled cavity therein. The casing includes an outer portion structured to sealingly engage a naris of a patient and an inner portion. The fill material is disposed in the sealed cavity defined by the casing and the base.

According to an example, a kit for providing a flow of gas to a patient is provided. The kit comprises a body member, a cushion and a plurality of nasal pillows. The body member comprises a pair of arms and a chin support, wherein a first end portion of each arm in the pair of arms is coupled to an opposite end of the chin support, and wherein the chin support is adapted to be disposed under the mandible responsive to the patient interface device being donned by a user. The body member further comprises at least one headgear attachment and a circuit portion operatively coupled to a second end portion of each arm in the pair of arms. The cushion includes a to the body member of the patient interface device. Each of the nasal pillows are adapted to be selectively coupled to the platform portion.

According to a further example, a method of providing a customizable patient interface device for providing a supply of gas to the naris of a patient is provided. The method comprises providing a patient interface device having a replaceable nasal pillow, the replaceable nasal pillow being structured to sealingly engage the naris of the patient. The method further comprises providing a kit including a plurality of nasal pillows. Each of the nasal pillows comprise a base portion, a casing and a fill material. The base portion is structured to be disposed on a portion of the patient interface device. The casing is coupled to the base in a manner which defines a sealed cavity therein. The casing includes an outer portion structured to sealingly engage a naris of a patient and an inner portion. The fill material is disposed in the sealed cavity defined by the casing and the base.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictales otherwise.
FIG. 1a is a front perspective view of a first embodiment of a patient interface device according to the principles of the present invention;
FIG. 1b is a front perspective view illustrating the positioning of the patient interface device of FIG. 1 a on a user;
FIGS. 2-4 are front, side, and rear views, respectively, of the patient interface device of FIG. 1a;
FIG. 5 is a front exploded view of the patient interface device of FIG. la;
FIG. 6 is a side view, of the body portion of the patient interface device of F1G. 5;
FIG. 7 is a cross-sectional view of an example of a replaceable nasal pillow installed on a portion of a corresponding platform;
FIG. 8 is an exploded cross-sectional view of the replaceable nasal pillow and platform of FIG. 7;
FIG. 9 is a cross-sectional view of another example of a replaceable nasal pillow installed on a portion of a corresponding platform;
FIG. 10 is a cross-sectional view of another example of a replaceable nasal pillow installed on a portion of a corresponding platform;
FIG. 11 is a cross-sectional view of another example of a replaceable nasal pillow installed on a portion of a corresponding platform;
FIG. 12 is an exploded view of the replaceable nasal pillow and platform of FIG. 11;
FIG. 13 is a partial cross-sectional view of another example of a replaceable nasal pillow installed on a portion of a corresponding platform;
FIG. 14 is an elevational view of the platform of FIG. 13;
FIG. 15 is a cross-sectional view of another example of a replaceable nasal pillow installed on a portion of a corresponding platform;
FIG. 16 is a cross-sectional view of another example of a nasal pillow to be used in conjunction with the platform of FIG. 15;
FIG. 17 is cross-sectional view of an example of a replaceable nasal pillow installed on a portion of a corresponding platform;
FIG. 18 is an isometric exploded view of the replaceable nasal pillow and platform of FIG. 17; and
FIGS. 19-20 are cross-sectional views of further example of a replaceable nasal pillow installed on a portion of a corresponding platform.

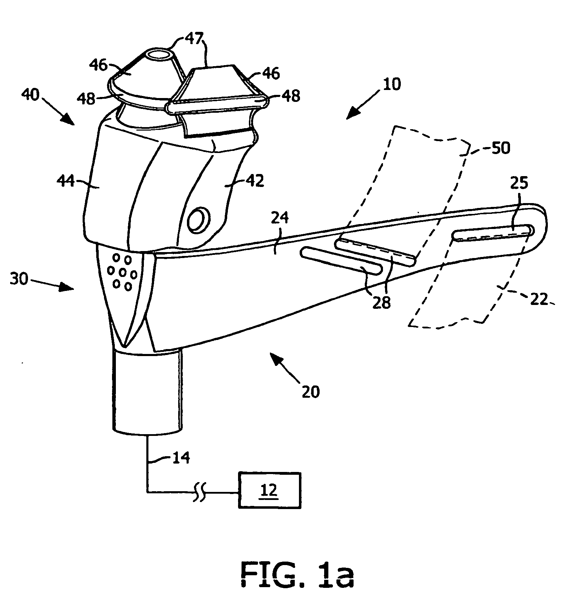

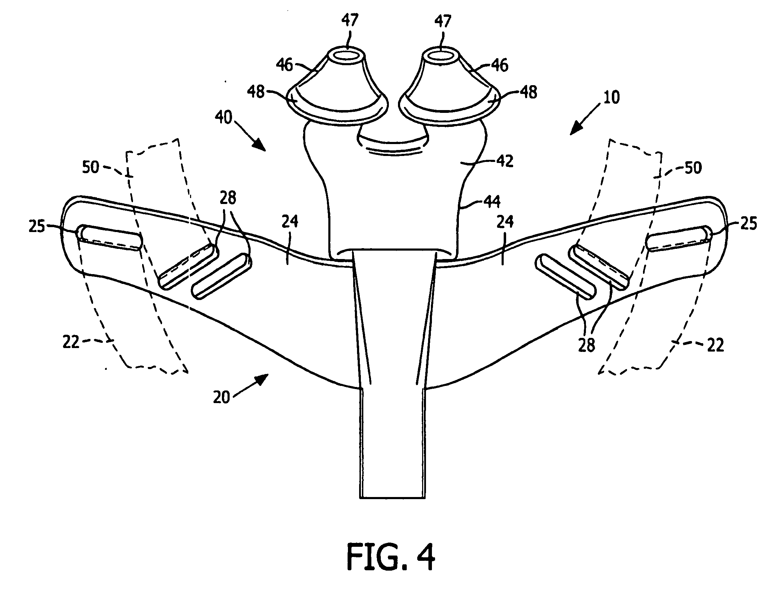

FIGS. 1a, 1b and 2-6 illustrate a general overview of an embodiment of the invention employed in conjunction with an example base member, such as described in U.S. Patent No. 7,500,480. It is to be appreciated that such base member 20 is provided for example purposes only as the present invention may be readily employed with a variety of base members and nasal cannula systems.

Referring to FIG. 1a, patient interface device 10 is shown schematically connected to a pressure support system 12 via a patient circuit 14, which communicates gas from the pressure support system 12 to the patient interface device. Patient circuit 14 is any device, such as a flexible tubing, that carries the flow of gas from the pressure/flow generator in the pressure support system to the patient interface device 10.

Pressure support system 12 is any conventional ventilation or pressure support system. Examples of such pressure support systems include, but are not limited to: a ventilator, continuous positive airway pressure (CPAP) device, or a variable pressure device, e.g. an auto-titrating device, proportional assist ventilation (PAV^{®}) device, proportional positive airway pressure (PPAP^{®}) device, C-Flex™ device, Bi-Flex™ device, or a BiPAP^{®} device manufactured and distributed by Respironics, Inc. of Pittsburgh, PA, in which the pressure provided to the patient varies with the patient's respiratory cycle so that a higher pressure is delivered during inspiration than during expiration, or other pressure support device.

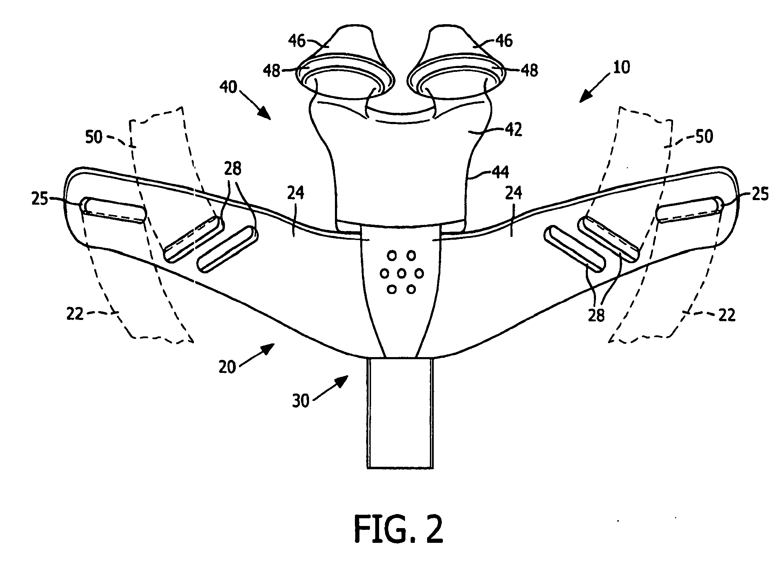

Continuing to refer to FIG. 1a, and additionally to FIGS. 2-4, patient interface device 10 includes a body portion 20, a circuit portion 30, and a patient interface portion 40. Body portion 20 includes a chin support 22 and a pair of arms 24. In the illustrated exemplary embodiment, circuit portion 30 and body portion 20 are integral with one another so that a gas flow path is defined through the patient interface device 10. Arms 24 include multiple headgear attachment elements 28 in the form of slots that are angled to match the angle of attachment of a headgear strap 50 when the patient interface device 10 is worn by the user. Attachment structures 25, also in the form of slots, are provided at the ends of arms 24. Chin support 22, which is a flexible strap, inserts through these slots. Typically, this chin strap is a padded strap that passes through the attachment structures 25 and loops back on itself. A hook and loop or other suitable fastener is generally provided on the strap so that the length of the strap may be adjusted.

Patient interface portion 40 includes a cushion 42 that attaches to circuit portion 30. Cushion 42 can be made from any suitable material, such as gel, silicone, foam, rubber, or a combination of materials and includes a base 44 and a pair of replaceable nasal pillows 46, each selectively coupled about an nasal aperture 47 in corresponding platforms 48 disposed generally along a top of cushion 42. As used here, the phrase "selectively coupled" shall be used to describe a coupling between at least two members such that the members may be uncoupled and coupled again without damaging either of the members. The structure of the replaceable nasal pillows 46 and cushion 42 will be discussed in further detail below in conjunction with a number of exemplary embodiments of the present invention.

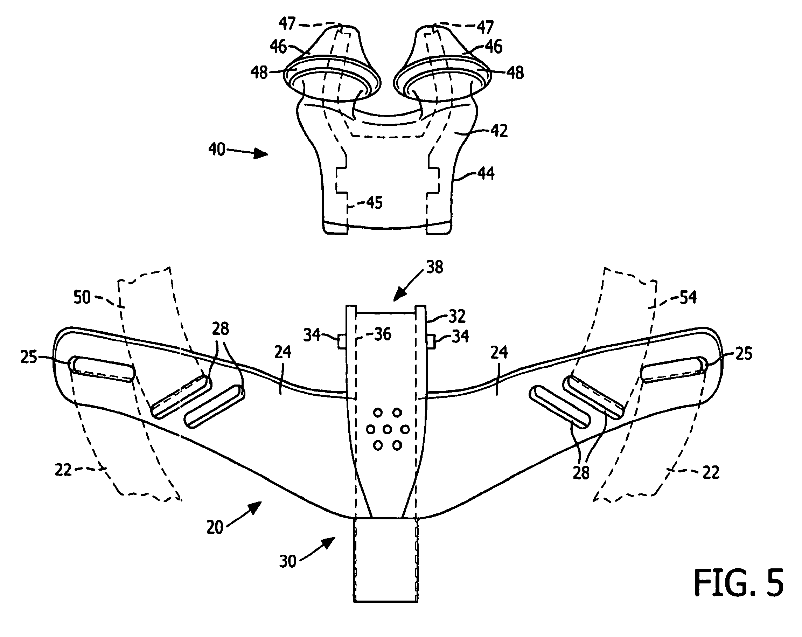

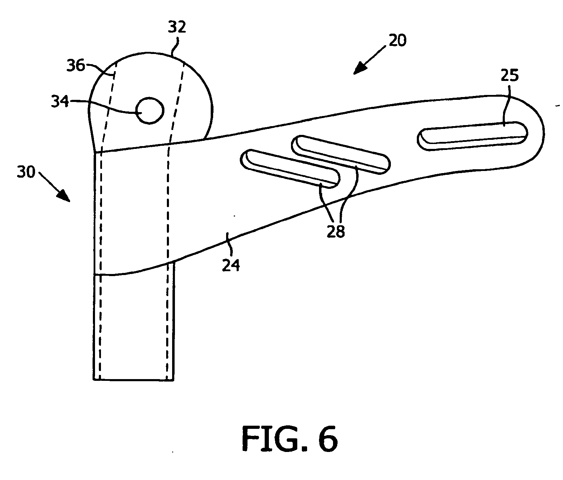

As best shown in FIGS. 5 and 6, circuit portion 30 includes a cushion attachment member 32 to which cushion 42 attaches in a male-female configuration. A cavity 45 (FIG. 5) is defined in base 44 of cushion 42 having a shape that generally matches the shape of cushion attachment member 32. As shown in FIG. 5, cavity 45 extends upward to each of nasal apertures 47. The cushion attaches to the circuit portion by sliding the base over cushion attachment member 32. The flexibility of the base 44 allows it to flex around the cushion attachment member 32 and the resiliency of the base 44 maintains the attachment.

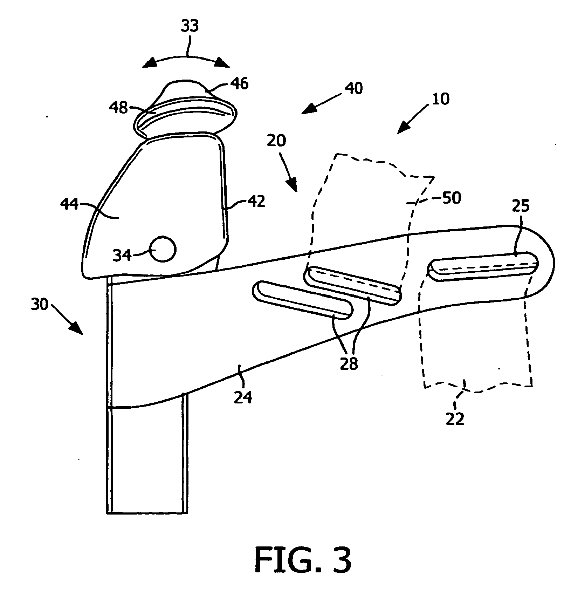

In the illustrated embodiment, cushion attachment member 32 has a barrel shape (and the cavity in the cushion has a similar barrel shape) so that the cushion can be moved or rotated relative to body portion 20 as indicated by arrow 33 in FIG. 3. The barrel shape, unlike a ball-and-socket, allows movement (rotation) in only one plane. Optional pins 34 can be provided on the sides (ends) of the barrel to help maintain the attachment between the cushion and the cushion attachment member 32 and allow the user to rotate the cushion about a fixed point and without dislodging it from the cushion attachment member. A passage 36 is defined through circuit portion 30, including through cushion attachment member 32. An opening 38 (FIG. 5) is defined in the top of cushion attachment member 32 so that gas flows between circuit portion 30 and cushion 42.

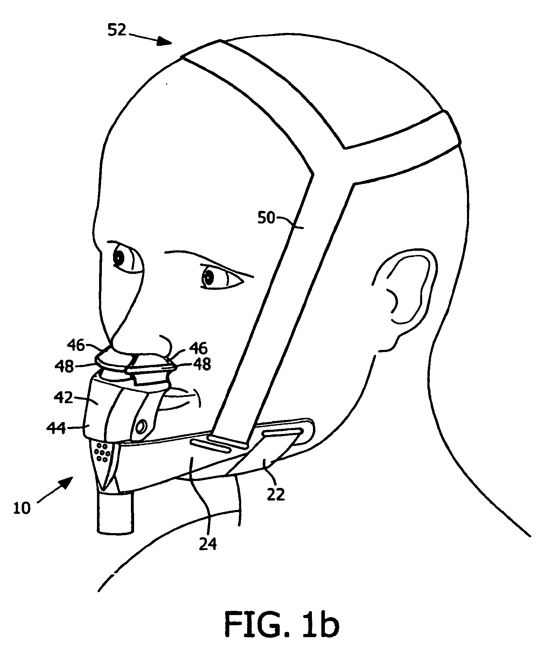

As shown in FIG. 1b, when patient interface device 10 is donned by the user, chin support 22 passes under the mandible from one side of the patients face to the other. A headgear assembly 52 including straps 50 maintain the patient interface device on the user. The two regions of the patient's face that primarily support the patient interface device on the user are the chin (via the chin support) and the patient's nasal region and/or nasal openings (via the patient interface portion) where each replaceable nasal pillow 46 sealingly engages a naris of the patient such that the flow of gas is provided to the patient from the pressure support system via patient circuit 14, circuit portion 30, and finally cavity 45 through nasal aperture 47.

It is to be appreciated that the modular nature of nasal pillows 46 (as well as the further embodiments thereof described below) provides for improved fitting of patient interface device 10 over known designs. Such improved fitting may be readily accomplished by individually selecting an appropriate nasal pillow 46 from among a selection of nasal pillows having varying characteristics. Such characteristics may include, for example without limitation, size, shape, relative harness, and surface texture. Such selection may be carried out by a medical caregiver or by a patient/user of the device 10. As each of the nasal pillows 46 are quite small in comparison to the overall patient interface device 10, much smaller packaging would be required to provide a plurality of such pillows to a patient as compared to a plurality of interface devices 10. As such, the present invention contemplates providing the user with a "fitting kit", including a variety of nasal pillows 46, thus allowing the user to custom fit a patient interface device 10. Additionally, a user may readily be supplied with a "customized" replacement kit having multiple copies of nasal pillows (46) to be used as replacements when those in use on a patient interface device wear out.

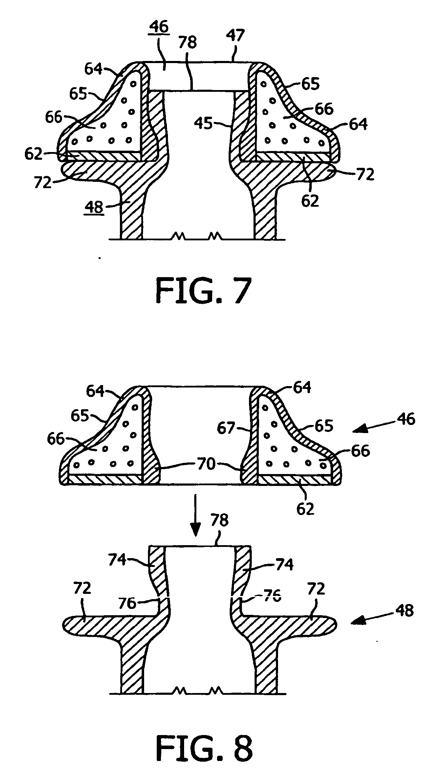

Having thus described a general overview of an example of a patient interface device 10, a first example of a replaceable nasal pillow 46 and corresponding platform 48 will now be described in conjunction with FIGS. 7 and 8 which show assembled and exploded cross-sectional views, respectively, of the combination. Nasal pillow 46 includes a generally flat, ring-like base member 62 and a casing 64 coupled to base member 62, to form a generally frusto-conical ring member defining a space (not numbered). Nasal pillow 46 further includes a fill material 66 disposed within the space formed by the assembly of base member 62 and casing 64. In the example depicted, casing 64, and similarly the further example thereof discussed herein, is formed from a generally thin flexible material (e.g., without limitation, silicone, polyurethane, thermal plastic elastomer, poly vinyl chloride, natural rubber) and includes a generally outer portion 65 and a generally inner portion 67.

Base member 62, as well as the further example thereof discussed herein, is generally formed from a similar material as casing 64 or a somewhat more rigid material such as a thermal plastic, e.g., without limitation, polycarbonate (PC), polypropylene (PP), and polystyrene (PS). Fill material 66 generally consists of a gel or other suitable material that generally conforms to the space defined by base 62 and casing 64 and allows for outer portion 65 of casing 64 to flex and conform to a nare of a patient. Inner portion 67 of casing 64 includes a thickened portion 70 that serves as a retention mechanism that cooperatively engages a corresponding retention mechanism (discussed below) of a platform 48 to selectively couple nasal pillow 46 to platform 48.

Continuing to refer to FIGS. 7 and 8, platform 48 includes a generally planar shelf portion 72 having a generally ring-like shape that encircles a generally tubular chimney portion 74 that extends upward from planar portion 72 and terminates at a top opening 78. Chimney portion 74 includes a reduced diameter portion 76 sized such that when nasal pillow 46 is installed on platform 48, thickened portion 70 of nasal pillow 46 cooperatively engages reduced diameter portion 76 (shown in FIG. 7) in a manner that selectively couples nasal pillow 46 to platform 48. It is to be appreciated that the relatively flexible nature of one or both of nasal pillow 46 and/or platform 48 allows for nasal pillow 46 to be "snapped" over opening 78 and onto platform 74 in a coupled position or "unsnapped" upon removal from platform 48 into an uncoupled position. It is contemplated that thickened portion 70 may extend along all (shown), or a portion of (not shown), inner portion 67 about chimney portion 74. Similarly, it is also contemplated that reduced diameter portion 76 may extend around all (shown), or selected portions of (not shown) chimney portion 74 as long as such reduced diameter portions 76 are provided, at minimum, in sufficient locations to cooperatively engage corresponding thickened portions 70 of nasal pillow 46.

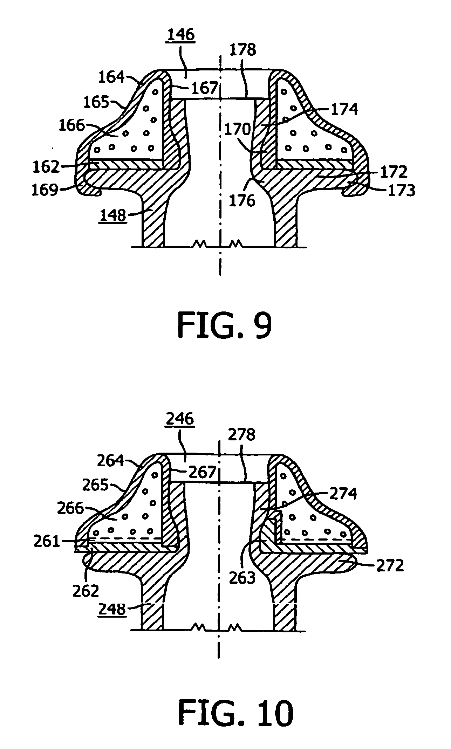

FIG. 9 illustrates a cross-sectional view of a second example of a replaceable nasal pillow 146 installed on a corresponding platform 148. Similar to the previous example, nasal pillow 146 includes a generally flat, ring-like base member 162 and a casing 164 coupled to base member 162, to form a generally frusto-conical ring member defining a space (not numbered) within. Nasal pillow 146 further includes a fill material 166 disposed within the space formed by the assembly of base member 162 and casing 164. In the example depicted, casing 164 is formed from a generally thin flexible material, such as previously discussed, and includes a generally outer portion 165 and a generally inner portion 167. Base member 162 is formed from a somewhat harder yet generally flexible material, such as previously discussed. Fill material 166 generally consists of a gel or other suitable material that generally conforms to the space defined by base 162 and casing 164 and allows for outer portion 165 of casing 164 to flex and conform to a nare of a patient. Inner portion 167 of casing 164 includes a thickened portion 170 that serves as a retention mechanism that cooperatively engages a corresponding retention mechanism (discussed below) of a platform 148 to selectively couple nasal pillow 146 to platform 148. Inner portion 167 of casing 164 includes a thickened portion 170 that serves as a retention mechanism that cooperatively engages a corresponding retention mechanism (discussed below) of a platform 148 to selectively couple nasal pillow 146 to platform 148.

Continuing to refer to FIG. 9, platform 148 includes a generally planar shelf portion 172 having a generally ring-like shape that encircles a generally tubular chimney portion 174 that extends upward from planar portion 172 and terminates at a top opening 178. Chimney portion 174 includes a reduced diameter portion 176 sized such that when nasal pillow 146 is installed on platform 148, thickened portion 170 of nasal pillow 146 cooperatively engages reduced diameter portion 176 in a manner that selectively couples nasal pillow 146 to platform 148. It is to be appreciated that the relatively flexible nature of one or both of nasal pillow 146 and/or platform 148 allows for nasal pillow 146 to be "snapped" over opening 178 and onto platform 174 in a coupled position or "unsnapped" upon removal from platform 148 into an uncoupled position. It is contemplated that thickened portion 170 may extend along all (not shown), or a portion of (shown), inner portion 167 about chimney portion 174. Similarly, it is also contemplated that reduced diameter portion 176 may extend around all (not shown), or selected portions of (shown) chimney portion 174 as long as such reduced diameter portions 176 are provided, at minimum, in sufficient locations to cooperatively engage corresponding thickened portions 170 of nasal pillow 146.

In addition to the cooperative engagement of thickened portion 170 and reduced diameter portion 176, the example depicted in FIG. 9 further includes an additional retention mechanism serving to selectively couple nasal pillow 146 to platform 148. Such additional retention mechanism is provided by a hooked portion 169 of casing 164 that extends below, and generally hooks around an end portion 173 of planar portion 172 when nasal pillow 146 is fully installed on platform 148.

FIG. 10 illustrates a cross-sectional view of a third example of a replaceable nasal pillow 246 installed on a corresponding platform 248. Similar to the previous example, nasal pillow 246 includes a generally flat, ring-like base member 262 and a casing 264 coupled to base member 262, to form a generally frusto-conical ring member defining a space (not numbered) within. Nasal pillow 246 further includes a fill material 266 disposed within the space formed by the assembly of base member 262 and casing 264. In the exemplary example depicted, casing 264 is formed from a generally thin flexible material and includes a generally outer portion 265 and a generally inner portion 267. Base member 262 is formed from a somewhat harder yet generally flexible material. Fill material 266 generally consists of a gel or other suitable material that generally conforms to the space defined by base 262 and casing 264 and allows for outer portion 265 of casing 264 to flex and conform to a nare of a patient. Base 262 may further be coupled to fill material 266 by an adhesive layer 261 in applications where a fill material 266 suitable for such bonding is employed. It is to be appreciated that such coupling between the base and fill material may further be employed with any of the example described herein when a suitable fill material is employed.

Base member 262 includes a vertical portion 263 adjacent inner portion 267 of casing 264 that serves as a retention mechanism that cooperatively engages a portion (discussed below) of a platform 248 to selectively couple nasal pillow 246 to platform 248.

Continuing to refer to FIG. 10, platform 248 includes a generally planar shelf portion 272 having a generally ring-like shape that encircles a generally tubular chimney portion 274 that extends upward from planar portion 272 and terminates at a top opening 278. Chimney portion 274 includes is generally sized such that when nasal pillow 246 is installed on platform 248, an interference fit is produced between chimney portion 274 and base member 262, particularly between chimney portion 274 and vertical portion 263 of base member 262. Such interference fit is provided in part due to the relative stiffness of base member 262 (and vertical portion 263 thereof) in comparison with the other materials of nasal pillow 246. It is to be appreciated that such interference fit allows for nasai piiiow 246 to be forcibly slid onto platform 248 in a coupied position or forcibly removed from platform 248 to be uncoupled. It is contemplated that vertical portion 263 of base member 262 may extend along all (not shown), or a portion of (shown), base member 262 about chimney portion 274.

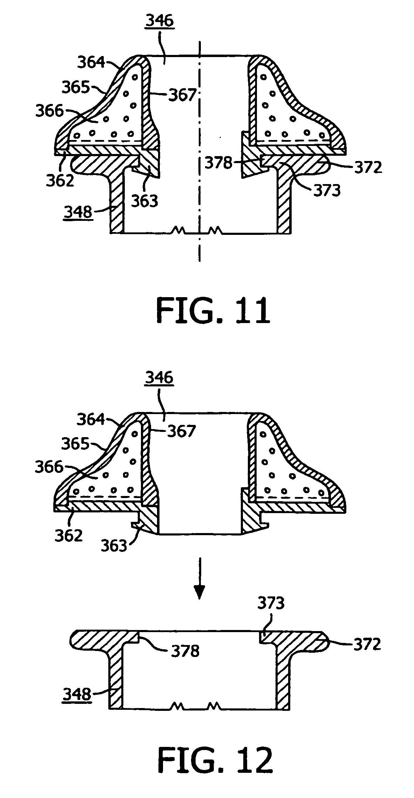

FIGS. 11 and 12 illustrate assembled and exploded cross-sectional views, respectively, of a fourth example of a replaceable nasal pillow 346 and corresponding platform 348. Nasal pillow 346 includes a generally flat, ring-like base member 362 and a casing 364 coupled to base member 362, to form a generally frusto-conical ring member defining a space (not numbered). Nasal pillow 346 further includes a fill material 366 disposed within the space formed by the assembly of base member 362 and casing 364. In the exemplary example depicted, casing 364 is formed from a generally thin flexible material and includes a generally outer portion 365 and a generally inner portion 367. Base member 362 is formed from a similar or somewhat harder yet generally flexible material. Fill material 366 generally consists of a gel or other suitable material that generally conforms to the space defined by base 362 and casing 364 and allows for outer portion 365 of casing 364 to flex and conform to a nare of a patient. Base portion 362 includes a downward facing hook portion 363 that serves as a retention mechanism that cooperatively engages a corresponding retention mechanism (discussed below) of a platform 348 to selectively couple nasal pillow 346 to platform 348.

Continuing to refer to FIGS. 11 and 12, platform 348 includes a generally planar shelf portion 372 having a generally ring-like shape. Planar shelf portion 372 includes an inward directed portion 373 defining an opening 378 sized such that when nasal pillow 346 is installed on platform 348, downward facing hook portion 363 of nasal pillow 346 cooperatively engages inward directed portion 373 of shelf portion 372 (FIG. 11) in a manner that selectively couples nasal pillow 346 to platform 348. It is to be appreciated that the relatively flexible nature of one or both of nasal pillow base 362 and/or planar shelf portion 372 allows for downward facing hook portion 363 of nasal pillow 346 to be "snapped" into opening 378 of platform 374, thus coupling nasal pillow 346 to platform 348, or "unsnapped" upon removal from platform 348 into an uncoupled position. It is contemplated that downward facing hook portion 363 may extend completely around (shown), or around a portion of (not shown), inner base portion 362. Similarly, it is also contemplated that inward directed portion 373 may extend inward from around all (shown), or selected portions of (not shown), planar shelf portion 372 as long as such inward directed portions 373 are provided, at minimum, in sufficient locations to cooperatively engage corresponding downward facing hook portions 363 of nasal pillow 346.

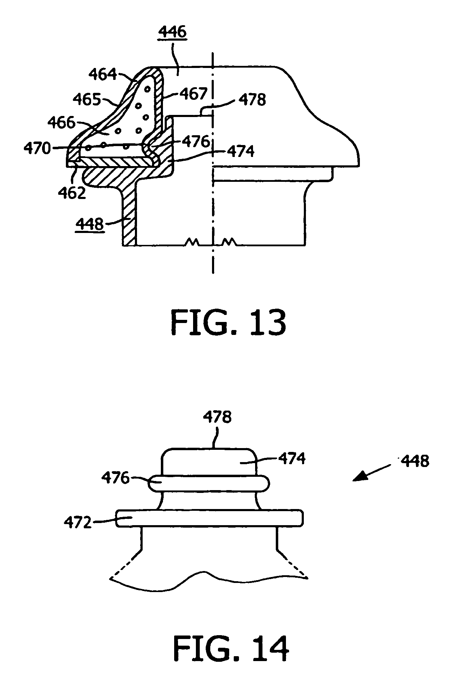

FIGS. 13 and 14 illustrate a partial cross-sectional view of a fifth example of a replaceable nasal pillow 446 installed on a corresponding platform 448 and an elevation view of platform 448, respectively. Nasal pillow 446 includes a generally flat, ring-like base member 462 and a casing 464 coupled to base member 462, to form a generally frusto-conical ring member defining a space (not numbered). Nasal pillow 446 further includes a fill material 466 disposed within the space formed by the assembly of base member 462 and casing 464. In the exemplary example depicted, casing 464 is formed from a generally thin flexible material and includes a generally outer portion 465 and a generally inner portion 467. Base member 462 is formed from a similar or somewhat harder yet generally flexible material such. Fill material 466 generally consists of a gel or other suitable material that generally conforms to the space defined by base 462 and casing 464 and allows for outer portion 465 of casing 464 to flex and conform to a nare of a patient. Inner portion 467 of casing 464 includes a grooved portion 470 that serves as a retention mechanism that cooperatively engages a corresponding retention mechanism (discussed below) of a platform 448 to selectively couple nasal pillow 446 to platform 448.

Continuing to refer to FIGS. 13 and 14, platform 448 includes a generally planar shelf portion 472 having a generally ring-like shape that encircles a generally tubular chimney portion 474 that extends upward from planar portion 472 and terminates at a top opening 478. Chimney portion 474 includes a rib portion 476 sized such that when nasal pillow 446 is installed on platform 448, grooved portion 470 of nasal pillow 446 cooperatively engages rib portion 476 in a manner that selectively couples nasal pillow 446 to platform 448. It is to be appreciated that the relatively flexible nature of one or both of nasal pillow 446 and/or platform 448 allows for nasal pillow 446 to be "snapped" over opening 478 and onto platform 474 in a coupled position or "unsnapped" upon removal from platform 448 into an uncoupled position. It is contemplated that grooved portion 470 may extend along all or a portion of inner portion 467 about chimney portion 474. Similarly, it is also contemplated that rib portion 476 may extend around all (shown), or selected portions of (not shown) chimney portion 474 as long as such grooved portions 470 are provided, at minimum, in sufficient locations to cooperatively engage corresponding rib portions 476 of chimney portion 474.

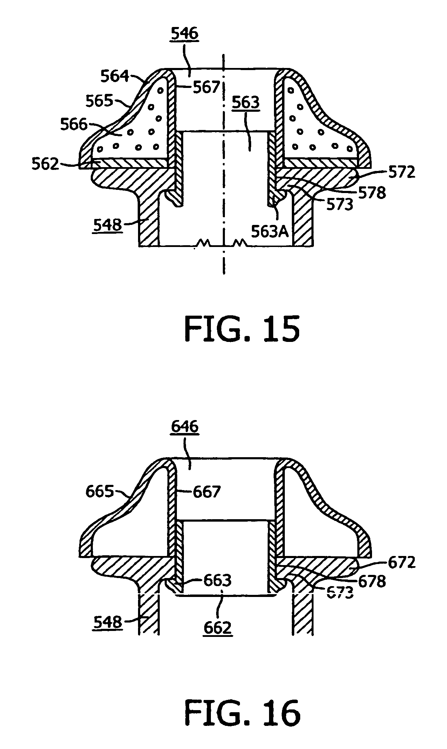

FIG. 15 illustrates a cross-sectional view of a sixth example of a replaceable nasal pillow 546 installed on a corresponding platform 548. Nasal pillow 546 includes a generally flat, ring-like base member 562 and a casing 564 coupled to base member 562, to form a generally frusto-conical ring member defining a space (not numbered) within. Nasal pillow 546 further includes a fill material 266 disposed within the space formed by the assembly of base member 562 and casing 564. In the example depicted, casing 564 is formed from a generally thin flexible material and includes a generally outer portion 565 and a generally inner portion 567. Base member 562 is formed from a similar or somewhat harder yet generally flexible material such as. Fill material 566 generally consists of a gel or other suitable material that generally conforms to the space defined by base 562 and casing 564 and allows for outer portion 565 of casing 564 to flex and conform to a nare of a patient.

Base portion of nasal pillow 546 further includes an inner member 563 of generally tubular shape coupled to inner portion 567 of casing 564. Inner member 563 extends downward from base member 562 and terminates in a hook portion 563A that serves as a retention mechanism that cooperatively engages a portion (discussed below) of a platform 548 to selectively couple nasal pillow 546 to platform 548.

Continuing to refer to FIG. 15, platform 548 includes a generally planar shelf portion 572 having a generally ring-like shape. Planar shelf portion 572 includes an inward directed portion 573 defining an opening 578 sized such that when nasal pillow 546 is installed on platform 548, hook portion 563A of inner member 563 cooperatively engages inward directed portion 573 of shelf portion 572 in a manner that selectively couples nasal pillow 546 to platform 548. It is to be appreciated that the relatively flexible nature of one or both of nasal pillow base 562 and/or planar shelf portion 572 allows for hook portion 563A of nasal pillow 546 to be "snapped" into opening 578 of platform 574, thus coupling nasal pillow 546 to platform 548, or "unsnapped" upon removal from platform 548 into an uncoupled position. It is contemplated that hook portion 563A may extend completely around (shown), or around a portion of (not shown), inner member 563. Similarly, it is also contemplated that inward directed portion 573 may extend inward from around all (shown), or selected portions of (not shown), planar shelf portion 572 as long as such inward directed portions 573 are provided, at minimum, in sufficient locations to cooperatively engage corresponding hook portions 563A of nasal pillow 546.

FIG. 16 illustrates a cross-sectional view of a seventh example of a replaceable nasal pillow 646 installed on a corresponding platform 648. Nasal pillow 646 includes a casing 664 forming a generally frusto-conical ring member defining a space (not numbered) below. In the example depicted, casing 564 is formed from a generally thin flexible material and includes a generally outer portion 665 and a generally inner portion 667. Outer portion 665 of casing 664 is generally configured to flex and conform to a nare of a patient.

Nasal pillow 646 further includes an inner member 662 of generally tubular shape coupled to inner portion 667 of casing 664. Inner member 662 extends downward from inner portion 667 and terminates in a hook portion 663 that serves as a retention mechanism that cooperatively engages a portion (discussed below) of a platform 648 to selectively couple nasal pillow 646 to platform 648.

Continuing to refer to FIG. 16, platform 648 includes a generally planar shelf portion 672 having a generally ring-like shape. Shelf portion 672 includes an inward directed portion 673 defining an opening 678 sized such that when nasal pillow 646 is installed on platform 648, hook portion 663 of inner member 662 cooperatively engages inward directed portion 673 of shelf portion 672 in a manner that selectively couples nasal pillow 546 to platform 648. It is to be appreciated that the relatively flexible nature of one or both of nasal inner member 662 and/or planar shelf portion 672 allows for hook portion 663 of nasal pillow 646 to be "snapped" into opening 678 of platform 674, thus coupling nasal pillow 646 to platform 648, or "unsnapped" upon removal from platform 648 into an uncoupled position. It is contemplated that hook portion 663 may extend completely around (shown), or around a portion of (not shown), inner member 662. Similarly, it is also contemplated that inward directed portion 673 may extend inward from around all (shown), or selected portions of (not shown), planar shelf portion 672 as long as such inward directed portions 673 are provided, at minimum, in sufficient locations to cooperatively engage corresponding hook portions 663 of nasal pillow 646.

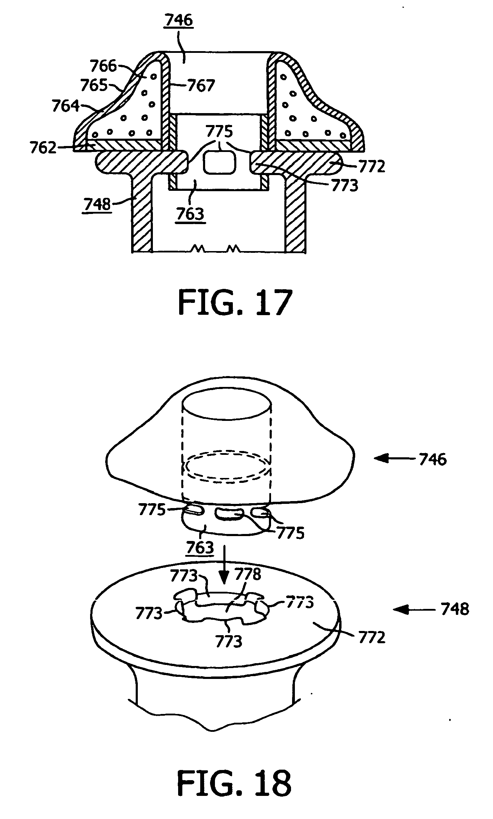

FIGS. 17 and 18 illustrate an assembled cross-sectional view and an isometric exploded view, respectively, of an eighth example of a replaceable nasal pillow 746 and corresponding platform 748 according to the principles of the present invention. Nasal pillow 746 includes a generally flat, ring-like base member 762 and a casing 764 coupled to base member 762, to form a generally frusto-conical ring member defining a space (not numbered). Nasal pillow 746 further includes a fill material 766 disposed within the space formed by the assembly of base member 762 and casing 764. In the example depicted, casing 764 is formed from a generally thin flexible material and includes a generally outer portion 765 and a generally inner portion 767. Base member 762 is formed from a similar or somewhat harder yet generally flexible material. Fill material 766 generally consists of a gel or other suitable material that generally conforms to the space defined by base 762 and casing 764 and allows for outer portion 765 of casing 764 to flex and conform to a nare of a patient.

Nasal pillow 746 further includes an inner member 763 of generally tubular shape coupled to inner portion 767 of casing 764. Inner member 763 extends downward from base member 762 and includes a number of apertures 775 that serve as a retention mechanism that cooperatively engages a portion (discussed below) of a platform 748 to selectively couple nasal pillow 746 to platform 748.

Continuing to refer to FIGS. 17 and 18, platform 748 includes a generally planar shelf portion 772 having a generally ring-like shape. Shelf portion 772 includes a number of protrusions 773 directed inward from shelf portion 772 toward an opening 778 that, when nasal pillow 746 is installed on platform 748, cooperatively engage each of the number of apertures 775 of nasal pillow 746 in a manner that selectively couples nasal pillow 746 to platform 748. It is to be appreciated that the relatively flexible nature of one or both of inner member 763 and/or planar shelf portion 772 allows for inner member 763 of nasal pillow 746 to be "snapped" into opening 778 of platform 774, thus coupling nasal pillow 746 to platform 748, or "unsnapped" upon removal from platform 748 into an uncoupled position. Although shown having four protrusions 773, it is to be appreciated that the number and relative sizing of such protrusions 773 may vary within the scope of the present invention.

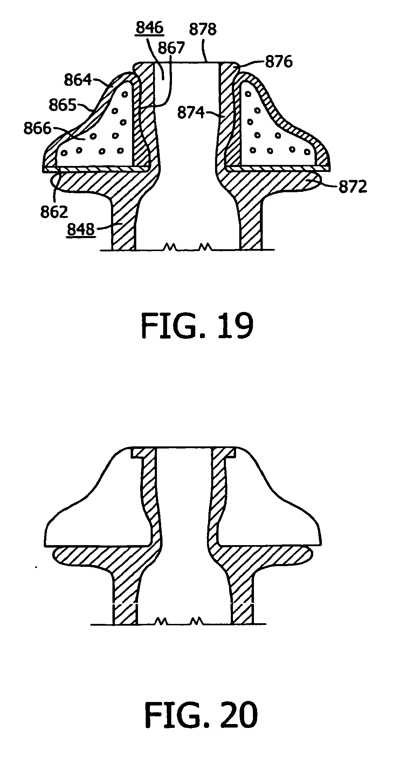

FIG. 19 illustrates a cross-sectional view of a ninth example of a replaceable nasal pillow 846 installed on a corresponding platform 848 according to the principles of the present invention. Nasal pillow 846 includes a generally flat, ring-like base member 862 and a casing 864 coupled to base member 862, to form a generally frusto-conical ring member defining a space (not numbered). Nasal pillow 846 further includes a fill material 866 disposed within the space formed by the assembly of base member 862 and casing 864. In the exemplary embodiment depicted, casing 864 is formed from a generally thin flexible material and includes a generally outer portion 865 and a generally inner portion 867. Base member 862 is formed from a similar or somewhat harder yet generally flexible material. Fill material 866 generally consists of a gel or other suitable material that generally conforms to the space defined by base 862 and casing 864 and allows for outer portion 865 of casing 864 to flex and conform to a nare of a patient.

Continuing to refer to FIG. 19, platform 848 includes a generally planar shelf portion 872 having a generally ring-like shape that encircles a generally tubular chimney portion 874 that extends upward from planar portion 872 and terminates at a top opening 878. Chimney portion 874 includes a rib portion 876 adjacent top opening 878 and sized such than when nasal pillow 846 is installed on platform 848, rib portion 876 engages an uppermost portion (not numbered) of inner portion 867 in a manner that selectively couples nasal pillow 846 to platform 848. It is to be appreciated that the relatively flexible nature of one or both of nasal pillow 846 and/or chimney portion 874 allows for nasal pillow 846 to be "snapped" over rib portion 876 and onto platform 874 in a coupled position or "unsnapped" upon removal from platform 848 into an uncoupled position. It is contemplated that rib portion 876 may extend about all (shown), or a portion of (not shown), tubular chimney portion 874.

## Claims

1. A kit for fitting a patient interface device (10) to the naris of a patient, the patient interface device employing a replaceable nasal pillow, the kit comprising:
a plurality of nasal pillows (46), wherein each nasal pillow is adapted to be selectively attached to a body portion of a patient interface device wherein each nasal pillow (46) of the plurality of nasal pillows comprises:
a base portion (62) structured to be disposed on such a body portion of the patient interface device;
and **characterized in that** each of the nasal pillows further comprises:
a casing (64) coupled to the base in a manner which defines a sealed cavity therein, the casing having an outer portion (65) structured to sealingly engage a naris of a patient and an inner portion (67); and
a fill material (66) disposed in the sealed cavity defined by the casing and the base portion.

2. The kit of claim 1, wherein each nasal pillow in the plurality of nasal pillows comprises a characteristic that varies from another nasal pillow in the plurality of nasal pillows.

3. The kit of claim 2, wherein the characteristic is selected from the group consisting: size, shape, hardness, and surface texture.

4. The kit of claim 1, wherein for each nasal pillow, the inner portion (67) of the casing comprises a thickened portion (70) that is structured to cooperatively engage a portion of the body portion of the patient interface device in a manner that selectively couples the nasal pillow to the body portion.

5. The kit of claim 4, wherein for each nasal pillow, the outer portion of the casing comprises a hooked portion (169) that is structured to extend below, and generally hook around a portion of the body portion of the patient interface device in a manner that selectively couples the nasal pillow to the body portion.

6. The kit of claim 1, wherein for each nasal pillow, the base portion comprises a vertical portion (263) adjacent the inner portion of the casing that is structured to engage as an interference fit a portion (274) of the body portion of the patient interface device in a manner that selectively couples the nasal pillow to the body portion.

7. The kit of claim 1, wherein for each nasal pillow, the base portion comprises a downward facing hook portion (363) that is structured to engage a portion of the body portion of the patient interface device in a manner that selectively couples the nasal pillow to the body portion.

8. The kit of claim 1, wherein for each nasal pillow, the inner portion of the casing comprises a grooved portion (470) that is structured to cooperatively engage a rib portion (476) of the body portion of the patient interface device in a manner that selectively couples the nasal pillow (446) to the body portion.

9. The kit of claim 1, wherein for each nasal pillow, the base portion comprises an inner member (563) of generally tubular shape coupled to the inner portion of the casing, the inner member extending downward from the base portion and terminating in a hook portion (563A) that is structured to cooperatively engage a portion of the body portion of the patient interface device in a manner that selectively couples the nasal pillow to the body portion.

10. The kit of claim 1, wherein for each nasal pillow, the base portion comprises an inner member (763) of generally tubular shape coupled to the inner portion of the casing, the inner member extending downward from the base portion and having an aperture (775) disposed therein that is structured to cooperatively engage a portion of the body portion of the patient interface device in a manner that selectively couples the nasal pillow to the body portion.

11. The kit of claim 1, wherein for each nasal pillow, an upper portion of the inner portion of the casing is structured to cooperatively engage a portion (876) of the body portion of the patient interface device in a manner that selectively couples the nasal pillow (846) to the body portion.

12. The kit of claim 1, further comprising a cushion (42) having a base portion (44) and a platform portion (48), the base portion being structured to couple the cushion to the body portion.

13. A system comprising:
- a patient interface for delivering a flow of breathing gas to a patient;
- the kit according to any of the preceding claims.

## Patentansprüche

1. Kit zur Anbringung einer Patientenschnittstellenvorrichtung (10) an der Naris eines Patienten, wobei die Patientenschnittstellenvorrichtung ein auswechselbares Nasenpolster nutzt, wobei das Kit Folgendes umfasst:
eine Vielzahl von Nasenpolstern (46), wobei jedes Nasenpolster dafür ausgelegt ist, selektiv an einem Körperteil der Patientenschnittstellenvorrichtung befestigt zu werden, wobei jedes Nasenpolster (46) der Vielzahl von Nasenpolstern Folgendes umfasst:
einen Basisteil (62), der strukturiert ist, um auf einem solchen Körperteil der Patientenschnittstellenvorrichtung angeordnet zu werden; und
**dadurch gekennzeichnet, dass** jedes der Nasenpolster weiterhin Folgendes umfasst:
ein Gehäuse (64), das mit der Basis auf eine Weise gekoppelt ist, die einen abgedichteten Hohlraum darin definiert, wobei das Gehäuse einen äußeren Teil (65), der strukturiert ist, um abdichtend in eine Naris eines Patienten einzugreifen, und einen inneren Teil (67) hat; und
ein Füllmaterial (66), das in dem durch das Gehäuse und den Basisteil definierten abgedichteten Hohlraum angeordnet ist.

2. Kit nach Anspruch 1, wobei jedes Nasenpolster der Vielzahl von Nasenpolstern eine Eigenschaft umfasst, die sich von einem anderen Nasenpolster der Vielzahl von Nasenpolstern unterscheidet.

3. Kit nach Anspruch 2, wobei die Eigenschaft ausgewählt wird aus der Gruppe bestehend aus: Größe, Form, Härte und Oberflächentextur.

4. Kit nach Anspruch 1, wobei für jedes Nasenpolster der innere Teil (67) des Gehäuses einen verdickten Abschnitt (70) umfasst, der strukturiert ist, um zusammenwirkend in einen Teil des Körperteils der Patientenschnittstellenvorrichtung auf eine Weise einzugreifen, die das Nasenpolster selektiv mit dem Körperteil koppelt.

5. Kit nach Anspruch 4, wobei für jedes Nasenpolster der äußere Teil des Gehäuses einen Hakenabschnitt (169) umfasst, der strukturiert ist, um sich unterhalb und im Allgemeinen als Haken um einen Teil des Körperteils der Patientenschnittstellenvorrichtung herum auf eine Weise zu erstrecken, die das Nasenpolster selektiv mit dem Körperteil koppelt.

6. Kit nach Anspruch 1, wobei für jedes Nasenpolster der Basisteil einen vertikalen Abschnitt (263) angrenzend an den inneren Teil des Gehäuses umfasst, der strukturiert ist, um in Presspassung in einen Teil (274) des Körperteils der Patientenschnittstellenvorrichtung auf eine Weise einzugreifen, die das Nasenpolster selektiv mit dem Körperteil koppelt.

7. Kit nach Anspruch 1, wobei für jedes Nasenpolster der Basisteil einen nach unten zeigenden Hakenabschnitt (363) umfasst, der strukturiert ist, um in einen Teil des Körperteils der Patientenschnittstellenvorrichtung auf eine Weise einzugreifen, die das Nasenpolster selektiv mit dem Körperteil koppelt.

8. Kit nach Anspruch 1, wobei für jedes Nasenpolster der innere Teil des Gehäuses einen mit einer Nut versehenden Abschnitt (470) umfasst, der strukturiert ist, um zusammenwirkend in einen Rippenteil (476) des Körperteils der Patientenschnittstellenvorrichtung auf eine Weise einzugreifen, die das Nasenpolster (446) selektiv mit dem Körperteil koppelt.

9. Kit nach Anspruch 1, wobei für jedes Nasenpolster der Basisteil ein inneres Element (563) von im Allgemeinen röhrenförmiger Form umfasst, das mit dem inneren Teil des Gehäuses gekoppelt ist, wobei sich das innere Element von dem Basisteil aus nach unten erstreckt und in einem Hakenabschnitt (563A) endet, der strukturiert ist, um zusammenwirkend in einen Teil des Körperteils der Patientenschnittstelle auf eine Weise einzugreifen, die das Nasenpolster selektiv mit dem Körperteil koppelt.

10. Kit nach Anspruch 1, wobei für jedes Nasenpolster der Basisteil ein inneres Element (763) von im Allgemeinen röhrenförmiger Form umfasst, das mit dem inneren Teil des Gehäuses gekoppelt ist, wobei sich das innere Element von dem Basisteil aus nach unten erstreckt und eine darin angeordnete Apertur (775) hat, die strukturiert ist, um zusammenwirkend in einen Teil des Körperteils der Patientenschnittstellenvorrichtung auf eine Weise einzugreifen, die das Nasenpolster selektiv mit dem Körperteil koppelt.

11. Kit nach Anspruch 1, wobei für jedes Nasenpolster ein oberer Teil des inneren Teils des Gehäuses strukturiert ist, um zusammenwirkend in einen Teil (876) des Körperteils der Patientenschnittstellenvorrichtung auf eine Weise einzugreifen, die das Nasenpolster (846) selektiv mit dem Körperteil koppelt.

12. Kit nach Anspruch 1, weiterhin umfassend ein Kissen (42) mit einem Basisteil (44) und einem Plattformteil (48), wobei der Basisteil strukturiert ist, um das Kissen mit dem Körperteil zu koppeln.

13. System, das Folgendes umfasst:
- eine Patientenschnittstelle zum Abgeben einer Atemgasströmung an einen Patienten;
- ein Kit nach einem der vorhergehenden Ansprüche.

## Revendications

1. Kit d'ajustement d'un dispositif d'interface patient (10) à la narine d'un patient, le dispositif d'interface patient employant un embout nasal remplaçable, le kit comprenant :
une pluralité d'embouts nasaux (46), dans lequel chaque embout nasal est à même d'être fixé sélectivement à une partie de corps d'un dispositif d'interface patient, dans lequel chaque embout nasal (46) de la pluralité d'embouts nasaux comprend :
une partie de base (62) structurée pour être disposée sur ladite partie de corps du dispositif d'interface patient ; et
**caractérisé en ce que** chacun des embouts nasaux comprend en outre :
une enveloppe (64) couplée à la base d'une manière qui y définit une cavité scellée, l'enveloppe ayant une partie externe (65) structurée pour s'engager de manière étanche dans une narine d'un patient et une partie interne (67) ; et
un matériau de remplissage (66) disposé dans la cavité scellée définie par l'enveloppe et la partie de base.

2. Kit selon la revendication 1, dans lequel chaque embout nasal de la pluralité d'embouts nasaux comprend une caractéristique qui varie de celle d'un autre embout nasal de la pluralité d'embouts nasaux.

3. Kit selon la revendication 2, dans lequel la caractéristique est choisie dans le groupe constitué de la taille, de la forme, de la dureté et de la texture de surface.

4. Kit selon la revendication 1, dans lequel, pour chaque embout nasal, la partie interne (67) de l'enveloppe comprend une partie épaissie (70) qui est structurée pour s'engager par coopération dans une portion de la partie de corps du dispositif d'interface patient d'une manière qui couple sélectivement l'embout nasal à la partie de corps.

5. Kit selon la revendication 4, dans lequel, pour chaque embout nasal, la partie externe de l'enveloppe comprend une partie à crochet (169) qui est structurée pour s'entendre en dessous et s'accrocher généralement autour d'une portion de la partie de corps du dispositif d'interface patient d'une manière qui couple sélectivement l'embout nasal à la partie de corps.

6. Kit selon la revendication 1, dans lequel, pour chaque embout nasal, la partie de base comprend une partie verticale (263) adjacente à la partie interne de l'enveloppe qui est structurée pour s'engager en ajustement serré sur une partie (274) de la partie de corps du dispositif d'interface patient d'une manière qui couple sélectivement l'embout nasal à la partie de corps.

7. Kit selon la revendication 1, dans lequel, pour chaque embout nasal, la partie de base comprend une partie de crochet (363) qui est tournée vers le bas et qui est structurée pour s'engager sur une portion de la partie de corps du dispositif d'interface patient d'une manière qui couple sélectivement l'embout nasal à la partie de corps.

8. Kit selon la revendication 1, dans lequel, pour chaque embout nasal, la partie interne de l'enveloppe comprend une partie rainurée (470) qui est structurée pour s'engager par coopération sur une partie nervurée (476) de la partie de corps du dispositif d'interface patient d'une manière qui couple sélectivement l'embout nasal (446) à la partie de corps.

9. Kit selon la revendication 1, dans lequel, pour chaque embout nasal, la partie de base comprend un élément interne (563) de forme générale tubulaire couplé à la partie interne de l'enveloppe, l'élément interne s'étendant vers le bas depuis la partie de base et se terminant par une partie à crochet (563A) qui est structurée pour s'engager par coopération sur une portion de la partie de corps du dispositif d'interface patient d'une manière qui couple sélectivement l'embout nasal à la partie de corps.

10. Kit selon la revendication 1, dans lequel, pour chaque embout nasal, la partie de base comprend un élément interne (763) de forme générale tubulaire couplé à la partie interne de l'enveloppe, l'élément interne s'étendant vers le bas depuis la partie de base et ayant une ouverture (775) qui y est ménagée et qui est structurée pour s'engager par coopération sur une portion de la partie de corps du dispositif d'interface patient d'une manière qui couple sélectivement l'embout nasal à la partie de corps.

11. Kit selon la revendication 1, dans lequel, pour chaque embout nasal, une portion supérieure de la partie interne de l'enveloppe est structurée pour s'engager par coopération sur une portion (876) de la partie de corps du dispositif d'interface patient d'une manière qui couple sélectivement l'embout nasal (846) à la partie de corps.

12. Kit selon la revendication 1, comprenant en outre un coussinet (42) ayant une partie de base (44) et une partie de plateforme (48), la partie de base étant structurée pour coupler le coussinet à la partie de corps.

13. Système comprenant :
- une interface patient pour délivrer un flux de gaz respiratoire à un patient ;
- le kit selon l'une quelconque des revendications précédentes.
